# EUROPEAN PATENT SPECIFICATION

(11) **EP 1 320 357 B1**
(45) Date of publication and mention of the grant of the patent: **19.11.2008**
(21) Application number: 01973825.1
(22) Date of filing: 27.09.2001
(51) Int. Cl.: A61K 9/107, A01N 25/10, A01N 25/02, A61P 31/04, A01N 25/04, A01N 25/24, A01N 35/02

(54) **TOPICAL POLYMERIC ANTIMICROBIAL EMULSION**
TOPISCHE POLYMERE ANTIMIKROBIELLE EMULSION
EMULSION ANTIMICROBIENNE POLYMERIQUE TOPIQUE

(30) Priority: 27.09.2000 AU PR040600
(43) Date of publication of application: 25.06.2003
(73) Proprietor: CHEMEQ LTD., Subiaco, WA 6008 (AU)
(72) Inventor: STATON, John, Alexander, Kingsgrove, New South Wales 2208 (AU); MELROSE, Graham, John, Hamilton, Dalkeith, Western Australia 6009 (AU)
(74) Representative: Tombling, Adrian George
(86) International application number: PCT/AU2001/001212
(87) International publication number: WO 2002/026211

(56) References cited:
- EP-A- 0 492 843
- EP-A- 0 526 267
- EP-A- 0 771 525
- WO-A-00/03723
- WO-A-00/61113
- WO-A-01/60874
- WO-A-91/17658
- WO-A-93/10209

## Description

### FIELD OF THE INVENTION

The present invention relates to topical emulsion compositions and to methods of preparing topical emulsion compositions.

### BACKGROUND TO THE INVENTION

Topical preparations such as dermatologicals, pharmaceuticals, sunscreens, cosmetics and topical biocides rely on a suitable a suitable carrier system to deliver the active components into the skin. The carrier typically consists of a mixture of one or more of the following ingredients: fats, oils, waxes and other hydrophobic substances, plus water, glycerol, propylene glycol and other hydrophilic substances, together with other pharmaceutically acceptable excipients such as preservatives, fragrances and colouring agents. The nature of the vehicle can play an important role in the efficacy or availability or rate and extent of release of the active agent from the delivery system.

Topically applied products usually contain water and other components in which microorganisms can grow. Such compositions generally contain preservatives which may be in the form of a single or combination of antimicrobial agents. Most antimicrobials are somewhat soluble in the aqueous components since microorganisms generally grow in the aqueous component. One group of antimicrobials which have been used are esters of p-hydrobenzoic acid known as parabens and include methyl, ethyl, propyl and butyl esters. Higher esters are also active but low solubility makes them less desirable to use.

There is presently increasing concern with regard to the safety of long term use of topical products. Possible adverse effects from transdermal absorption of organic chemical components of the vehicle are also of particular concern. For instance, the use of parabens as a preservative may lead to adverse reactions following transdermal absorption in sensitive individuals. Furthermore compounds used in combination with parabens such as imidazolidinyl urea or diazolidinyl urea may cause skin irritation in some people. Further, aromatic amine derivatives used in many sunscreen preparations may cause irritation or allergy. It is difficult to maintain emulsion stability and effective dispersion of inorganic components in a composition.

International application WO 00/03723 discloses a method for the preparation of compositions of poly(2-propenal, 2-propenoic acid). In Example 8, the effects of the presence of the polymer on the migration of various agents is studied. WO 00/03723 is concerned with providing a polymer which is stable in aqueous solution but does not disclose a composition with a polymeric oil phase.

European patent application EP-A-0526276 relates to polyfluoroalkylthiopoly(ethylimidazolium) compounds and their use as biocidal agents.

It is one object of the present invention to provide a topical composition which substantially overcomes the abovementioned problems associated with the prior art, or at least provides a useful alternative thereto.

It is a further object of the present invention to provide a sunscreen composition with solid active agents such as titanium dioxide or zinc oxide or their derivatives whilst still maintaining the sunscreening properties of the active agent.

It is a further objective to provide an antiseptic composition which is stable and of low irritancy.

### SUMMARY OF THE INVENTION

In accordance with the present invention there is provided an emulsion composition for topical application comprising:
(a) 10 to 60% by weight of polymeric oil phase having an average molecular weight of at least 1000;
(b) 1 to 15% by weight of polymeric emulsifier component of average molecular weight of at least 1000;
(c) 30 to 90% by weight of an aqueous phase; and
(d) at least 0.005% by weight of poly(2-propenal, 2-propenoic acid) polymeric antimicrobial said polymeric antimicrobial having an average molecular weight of at least 1000,
wherein at least 95% by weight of the organic components of the composition have an average molecular weight of at least 1000.

Optionally, the composition further comprises one or more components selected from polymeric film formers, inorganic or polymeric stabilizers, other inorganic or polymeric excipients and mixtures thereof. Preferably substantially all the organic components of the topical composition are polymeric.

The composition preferably comprises an active agent, which may be a therapeutic agent. The active agent may be a solid sunscreen agent such as a microfine particulate metal oxide. Zinc oxide and titanium dioxide and mixtures thereof are preferred. The composition of the invention comprises a sufficient amount of antimicrobial to provide antiseptic properties on the skin.

In accordance with the present invention there is further provided a process for preparing an emulsion composition comprising dissolving a poly(2-propenal, 2-propenoic acid) in an aqueous solution, mixing the aqueous phase with a polymeric oil phase in the presence of a polymeric emulsifier to provide an emulsion composition according to claim 1.

Optionally, the aqueous solvent may contain one or more inorganic or polymeric stabilizers. Further, the aqueous phase may be warmed up to about 90°C.

Optionally the polymeric hydrophobic phase may contain one or more active agents. Further, the polymeric hydrophobic phase may be warmed up to about 90°C.

There is still further provided the use of compositions as described hereinabove for purposes of topical or dermatological application in humans or animals.

### DETAILED DESCRIPTION OF THE INVENTION

The present invention provides an emulsion composition for the delivery of an antimicrobial agent intended for topical application. The carrier components of the composition are specifically chosen polymers. As such, the components of the carrier will be inhibited from being absorbed transdermally into the vascular circulatory system and consequently, higher safety will result.

Optionally, the composition further comprise one or more polymeric film formers and/or one or more inorganic or polymeric stabilizers and/or other inorganic or polymeric excipients.

The composition may comprise one or more active agents, which may be a therapeutic agent. The active agent may further be an inorganic sunscreen agent, including zinc oxide or titanium dioxide or derivatives or combinations thereof, or other inorganic solids capable of performing a sun-screening function.

The emulsion composition of the invention comprises sufficient polymeric antimicrobial to provide antiseptic action on skin.

The emulsion compositions of the invention comprise a polymeric oil phase. The oil phase may for example include polyalkylene glycols, organosilane polymer (polycarbosilanes), polyvinyl alcohols, polyvinyl esters, polyvinylpyrrolidones, alkoxypolyethylene glycols, polysilanes, styrene-acrylate copolymers, polysaccharides, copolymers thereof and mixtures thereof. Preferred oil phase components are selected from the group consisting of polyorganosiloxanes.

Polyorganosiloxanes are particularly preferred as we have found them to provide emulsion stability in the polymeric compositions of the invention. The oil component is preferably free of fatty and paraffinic components. Polyorganosiloxanes include polyalkylsiloxanes and polyalkylaryl siloxanes. Examples of polyalkylene glycols include polyethylene glycol, polypropylene glycol and copolymers of ethylene glycol and propylene glycol (ethylene oxide and propylene oxide).

The average molecular weight of the oil phase component or each of the oil phase components is typically at least 1000.

Specific examples of the oil phase include cyclomethicone, imethicone, dimethicone copolyol and bis-phenylpropyl dimethicone. The concentration of the polymeric oily phase is 10% to 60% by weight of the composition with a preferred concentration of 15% to 50% by weight of the composition.

The polymeric emulsifier preferably comprises a polyoxyalkylene portion Examples of emulsifiers include hexitan esters such as polysorbates, polyethoxylated alkyl phenols, polyethoxylated fatty alcohols, poloxamers, polyoxyethylene glycol monoethers, alkyl polyglucosides and ethoxylated polysiloxanes. The polymeric emulsifier is from 1 to 15% by weight of the total composition and preferably from 1 to 11% by weight. The optimum surfactant composition will depend on the nature of the oil phase. It is particularly preferred that the emulsifier component comprise a primary emulsifier comprising one or more ethoxylated polysiloxanes and a secondary emulsifier comprising one or more emulsifiers selected from the group of polyoxyalkylene emulsifiers such as one or more selected from polysorbates, polyethoxylated alkylphenols, polyethoxylated fatty alcohols, poloxamers, polyalkylene glycol monoethers and alkyl polyglucosides.

The secondary emulsifier is most preferably selected from polyethylene glycol monoethers or mixture thereof. Polyethylene glycol monoether emulsifiers are commercially available as the "Brij" series and include polyoxyethylene lauryl ethers (Brij 30, 35) cetyl ethers (Brij 52, 56, 58), stearyl ethers (Brij 72, 76, 78) and oleyl ethers (Brij 92, 96, 98). The number average molecular weight of each of the emulsifier components is at least 1000.

Specific examples of the most preferred polymeric primary emulsifier are ethoxylated polylaurylmethicone copolyol emulsifier, sodium PG propyldimethicone thiosulphate copolymer and cetyl dimethicone copolyol. The concentration of the primary emulsifier is typically 1% to 10% by weight of the composition, preferably 2% to 8% by weight of the composition and most preferably 4% to 8% by weight of the composition.

Specific examples of the most preferred polymeric secondary emulsiflers include polyethylene glycol ethers such as steareth 20 and polyethoxylated fatty alcohols such as laureth 23. The concentration of the secondary emulsifier is 0.05% to 5% by weight of the composition but preferably 0.2% to 1% by weight of the composition.

The concentration of the aqueous phase is 30% to 90% by weight of the composition, preferably 50% to 80% by weight of the composition.

The polymeric preservative of the invention is poly(2-propenal,2-propenoic acid) which is selected from polymers and of acrolein and mixtures thereof.

The preferred acrolein polymers and/or copolymers have the polymeric repeating unit: 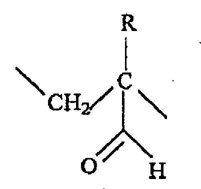 wherein R is H or alkyl, usually C₁ to C₄ or this unit is hydrated, hemiacetal or acetal form and illustrated non-comprehensively of all possible structures, by the following formulae: 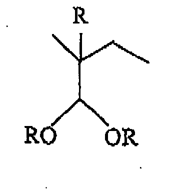 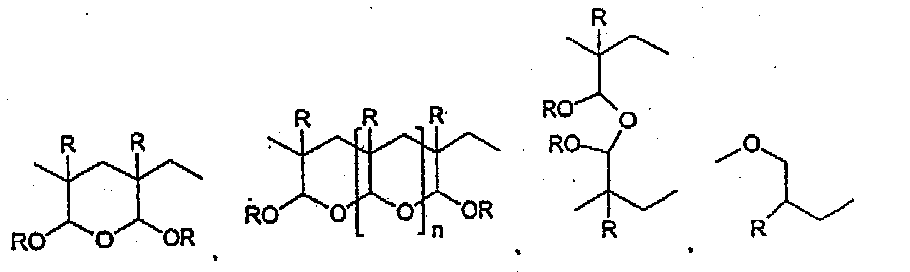 or 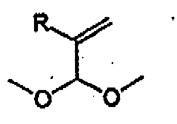 wherein n is one or more and R is as defined above or may be a polyether chain such as a polyethylene glycol. Examples of acrolein polymers and copolymers are described in US Patent 5290894 (Melrose et al).

Preferably the polymeric preservatives is an acrolein polymer of the type described in our International Applications PCT/AU96/00328 (WO 96/38186) and more preferably is a super activated acrolein polymer of the type described in PCT/AU00/00107 (WO 01/60874) which belongs to the prior art persuant to Article 54(3) EPC. Acrolein polymers of this type comprise poly(2-propenal, 2-propenoic acid) and are prepared by oxidation of solid polymer (poly-2-propenal) in air.

The activated acrolein polymers described in WO 96/38186 and WO 01/60874 are particularly suited to use in compositions as they are generally stable in the formulation and are sufficiently water soluble to provide preservative and antiseptic properties. They are also safe as the polymers are not absorbed across the skin barrier. The polymers of WO 01/60874 exhibit enhanced microbial activity as a result of heating of poly(2-propenal, 2-propenoic) acid in a solution of polyethylene glycol, polyol or alkanol containing water. Super-activation is facilitated by the presence of polyethylene glycols or polyols or alkanols. We believe that the presence of the polyethylene glycol or polyol or alkanol protects and stabilizes the carbonyl groups of the polymers, possibly by formation of acetals, from alkaline degradation by the Cannizarro reaction.

An added advantage of super-activation is that it reduces significantly the presence of contaminant acrolein which is a source of tissue and dermal irritation.

Suitable acrolein polymers are available from Chemeq Limited under the trade mark CHEMYDE. The average molecular weight of each of the preservative components is at least 1000. The concentration of the polymeric preservative is at least 0.005% by weight of the composition, preferably 0.01% to 1.0% by weight of the composition, and more preferably 0.05% to 0.4% by weight of the composition.

Examples of the polymeric film former include but are not restricted to PVP/Polycarbamylpolyglycol ester, PVP/eicosene copolymer, PVP/dimethiconylacrylate/polycarbamyl/polyglycol ester. The concentration of the film former is 0.5% to 5% by weight, preferably 1% to 3% by weight of the emulsion composition.

The composition can be a sunscreen composition containing a sunscreen agent such as a particulate metal oxide. Titanium dioxide and zinc oxide are particularly preferred. The concentration of metal oxide in the composition will typically be in the range of from 0.5 to 50% by weight and preferably from 1 to 40% by weight of the composition.

In an alternative embodiment the composition is a topical antiseptic. In the composition for use as an antiseptic the concentration of polymeric preservative will be sufficient to provide an antiseptic activity when applied to skin.

When the polymeric preservative is an acrolein polymer or copolymer such as the CHEMYDE brand antimicrobial the concentrations for use in an antiseptic is typically at least 1% by weight and more preferably at least 2% by weight.

At least 95% by weight of the organic components of the emulsion composition have a number average molecular weight of at least 1000 and more preferably at least 98% of the organic components have a number average molecular weight of at least 1000. Most preferably 100% of the organic components have a number average molecular weight of at least 1000.

The use of organic components of molecular weight of over 1000 reduces significantly the problem of irritation and transdermal transmission associated with the prior art. This is especially so when the proportion of the total organic components of molecular weight over 1000 is very high. Furthermore it also produces an emulsion of high stability particularly when the preferred polymeric components described above are used.

The composition of the invention may be in the form of a water in oil emulsion or an oil in water emulsion. Oil in water emulsions are generally preferred.

In the process according to the invention the polymeric preservative is dissolved in aqueous solvent to provide an aqueous phase. When the polymeric preservative is an acrolein polymer or copolymer, particularly a super activated poly(2-propenal, 2-propenoic acid), it is generally more convenient to dissolve the polymer in mildly alkaline solution (such as dilute aqueous carbonate solution) as dissolution occurs more readily in alkaline solution. The pH of the aqueous phase may then be adjusted by addition of acid if desired.

The poly(2-Propenal, 2-propenoic acid) polymer is typically soluble in water in an amount of from 0.5 to 5% at a pH of from 6 to 8 at 25°C. The solubility of the polymer typically increases with an increasing pH above 7. The pH of the aqueous phase is typically in the range of from 6 to 8.

The aqueous phase is combined with the oil phase with vigorous mixing in the presence of the emulsifier to provide an emulsion. Additional components may be present during formation of the emulsion or may be subsequently added.

The present invention will now be described with reference to the following examples which are not to be interpreted to limit the scope of the present invention.

### Example 1

An example of the composition of the invention when used as a sunscreen emulsion is shown below. Super activated polyacrolein polymer was prepared in accordance with Example 1 of International Application PCT/AU00/00107 (WO 01/60874). The resulting polymer is a poly(2-propenal, 2-propenoic acid) polymer super activated by heating in aqueous polyol solution such as PEG. The PEG is believed to provide a derivative with protected carbonyl groups possibly by formation of acetals by a Cannizarro reaction. The polymer was dissolved in the aqueous phase.

| **Raw Material** | **Function** | **% w/w** |
|---|---|---|
| Purified water | Aqueous phase | 49.5 |
| VEEGUM Ultra | Inorganic stabilizer (magnesium aluminium silicate) | 1.0 |
| Sodium Chloride | Inorganic stabilizer | 2.0 |
| CHEMYDE^{RTM} antimicrobial | Polymeric preservative | 0.2 |
| Antaron V220 | Polymeric film former | 2.0 |
| BRIJ 78 | Polymeric secondary emulsifier | 0.3 |
| DCQ2-5200 | Polymeric primary emulsifier | 5.0 |
| GE SF 1555 Bis phenylpropyl dimethicone | Polymeric hydrophobic component | 15.0 |
| Titanium Dioxide | Inorganic sunscreen | 13.0 |
| Zinc Oxide | Inorganic sunscreen | 2.0 |
| DC silicone 1401 | Polymeric excipient (emollient) | 5.0 |
| SUNSPHERES | Styrene-acrylate copolymer (polymeric hydrophobic component) | 5.0 |

Samples of the sunscreen, after accelerated ageing at 80°C for 4 months maintained emulsion stability and a sun protection factor of 15, and were inoculated after storage with different bacteria in accordance with the Preservative efficiency test BP Topical (EP 2000; Method TM107). The bacterial population after storage at 20°C for various periods is shown in Table 1 below:

**Table 1**

| Time Point | *S. aureus* AMS 027 (ATCC6538) | *P. aeruginosa* AMS 017 (ATCC9027) |
|---|---|---|
| Inoculum CFU/ml | 3.8 x 10⁵ | 5.2 x 10⁵ |
| 0 hr | 5.9 x 10⁵ | 1.4 x 10⁵ |
| 48 hr | <100 | <100 |
| 7 Days | <100 | <100 |
| 14 Days | <100 | <100 |
| 28 Days | <100 | <100 |

| | | |
|---|---|---|
| All results are expressed as CRU Colony Forming Unit) per g. < less than | | |

The method used in the preparation of the above composition was as follows:
1. Prepare the aqueous phase
   1.1 Disperse the VEEGUM magnesium aluminium silicate (inorganic stabilizer) in de-ionized water by stirring for 20 minutes, and then add the sodium chloride, stirring until dissolved;
   1.2 Adjust the pH to slightly basic (pH 7.5) and then add the CHEMYDE^{RTM} antimicrobial polymer (polymeric preservative) with stirring, until dissolved;
   1.3 Heat to 70°C.
2. Separately, prepare the polymeric oily phase:
   2.1 Heat a mixture of the ANTARON PVP/Eicosene copolymer (polymeric film former), BRIJ78 steareth-20 (polymeric secondary emulsifier), DCO2-5200 laurylmethicone copolymer (polymeric primary emulsifier), and bis phenylpropyl dimethicone (polymeric hydrophobic component), with stirring, to 70°C,
   2.2 Add the titanium dioxide and zinc oxide, with stirring, to disperse in the above oily phases, whilst maintaining the temperature at 70°C.
3. Mix to emulsify the aqueous and polymeric oily phases:
   3.1 Very slowly add the oily phase (from 2.2) to the aqueous phase (from 1.3) whilst stirring with a Silverson mixer at 5,000 rpm, until emulsified.
4. Whilst continuing to stir, add the DC SILICONE FLUID 1401 cyclomethicone and dimethiconol (polymeric emollient).
5. Allow to cool to 50°C, then add the "Sunspheres" using slow speed stirring.

### Example 2

The following formulation of an emollient hand cream is prepared by the same method as Example 1:

| **Raw Material** | **Function** | **% w/w** |
|---|---|---|
| Purified water | Aqueous Phase | 69.5 |
| VEEGUM Ultra | Inorganic stabilizer | 1 .0 |
| Sodium Chloride | Inorganic stabilizer | 2.0 |
| CHEMYDE^{RTM} antimicrobial | Polymeric preservative | 0.2 |
| Antaron V 200 | Polymeric film former | 2.0 |
| BRIJ 78 | Polymeric secondary emulsifier | 0.3 |
| DCQ2-5200 | polymeric primary emulsifier | 5.0 |
| GE Silicone fluid SF1555 | Polymeric hydrophobic component and emollient | 15.0 |
| DC Silicone fluid 1401 | Polymeric emollient | 5.0 |

The produce was aged at 40°C for 4 months after which it was stable and passed the BP challenge by bacteria as described in Example 1.

### Example 3

This example demonstrates a composition of the invention in the form of an antimicrobial antiseptic lotion which may be used to sanitize and protect skin from undesirable bacterial levels:

| **Raw Material** | **Function** | **% w/w** |
|---|---|---|
| Purified water | Aqueous Phase | 67.7 |
| VEEGUM Ultra | Inorganic stabilizer | 1 .0 |
| Sodium Chloride | Inorganic stabilizer | 2.0 |
| CHEMYDE^{RTM} antimicrobial | Polymeric preservative and sanitizing agent | 2.0 |
| Antaron V200 | Polymeric film former | 2.0 |
| BRIJ 78 | Polymeric secondary emulsifier | 0.3 |
| DCQ2-5200 | polymeric primary emulsifier | 5.0 |
| Bis phenylpropyl dimethicone | Polymeric hydrophobic component | 15.0 |
| DC silicone 1401 | Polymeric excipient (emollient) | 5.0 |

The produce was aged at 40°C for 4 months after which it was stable and passed the BP challenge by bacteria as described in Example 1.

The compositions of the present invention provide a vehicle for the topical delivery of an antimicrobial agent of which substantially overcome the problems associated with the prior art, or at least provides a useful alternative thereto. Further, the vehicle is safe, since the vehicle, in having all or substantially all organic components, polymeric, is not significantly absorbed through the skin via the transdermal route.

## Claims

1. An emulsion composition for topical application comprising:
(a) 10 to 60% by weight of polymeric oil phase having an average molecular weight of at least 1000;
(b) 1 to 15% by weight of polymeric emulsifier component of average molecular weight of at least 1000;
(c) 30 to 90% by weight of an aqueous phase; and
(d) at least 0.005% by weight of poly(2-propenal, 2-propenoic acid) polymeric antimicrobial said polymeric antimicrobial having an average molecular weight of at least 1000,
wherein at least 95% by weight of the organic components of the composition have an average molecular weight of at least 1000.

2. An emulsion composition according to claim 1 wherein the poly(2-propenal, 2-propenoic acid) has been activated by heating in an aqueous solution of one or more polyethylene glycols, polyols and alkanols.

3. An emulsion composition according to claim 1 wherein the polymeric oil phase comprises one or more polymers selected from the group consisting of polyalkylene glycols, organosilane polymer (polycarbosilanes), polyvinyl alcohols, polyvinyl esters, polyvinylpyrrolidones, alkoxypolyethylene glycols, polysilanes, styrene-acrylate copolymer, polysaccharides, copolymers thereof and mixtures thereof.

4. An emulsion composition according to claim 1 wherein the polymeric oil phase comprises one or more polymers selected from the group consisting of polyorganosiloxanes.

5. An emulsion composition according to claim 1 wherein the polymeric emulsifier includes one or more emulsifiers selected from the group of polyoxyalkylene emulsifiers and ethoxylated polysiloxane emulsifiers.

6. An emulsion composition according to claim 1, wherein the polymeric emulsifier comprises a primary emulsifier comprising one or more ethoxylated polysiloxane emulsifiers and a secondary emulsifier comprising one or more polyoxyalkylene emulsifiers.

7. An emulsion composition according to claim 5, wherein the primary emulsifier comprises one or more emulsifiers selected from the group consisting of ethoxylated polyacrylmethicone copolymer, sodium PG propyldimethicone thiosulphate copolymer and cetyl dimethicone copolyol.

8. An emulsion composition according to claim 5, wherein the secondary emulsifier comprises one or more emulsifiers selected from the group consisting of polyethylene glycol ethers and polyethoxylated fatty alcohols.

9. An emulsion composition according to claim 5, wherein the primary emulsifier is present in an amount of from 1 to 10% by weight of the composition and the secondary emulsifier is present in an amount of from 0.5 to 5% by weight of the composition.

10. An emulsion composition according to claim 1 wherein the composition includes from 0.5 to 5% of a polymeric film former.

11. An emulsion composition according to claim 10 wherein the polymeric film former is selected from the group consisting of PVP/Polycarbomoylpolyglycol ester, PVP/eicosene copolymer, PVP/dimethiconylacrylate/polycarbamyl/polyglycol ester, and mixtures thereof.

12. An emulsion composition' according to claim 1, wherein the composition comprises an inorganic or polymeric stabiliser.

13. An emulsion composition according to claim 12, wherein the stabiliser is magnesium aluminium silicate.

14. An emulsion composition according to claim 1 for use as a sunscreen and further comprising at least one particulate sunscreen agent selected from zinc oxide and titanium dioxide.

15. An emulsion composition according to claim 14 wherein the particulate sunscreen agent is present in an amount of from 0.5% to 50% by weight of the composition.

16. An emulsion composition according to claim 15 wherein the composition comprises a poly(2-propenal, 2-propenoic acid) in an amount of from 0.005 to 2% by weight.

17. An emulsion composition according to claim 1 for use as a skin antiseptic comprising poly(2-propenal, 2-propenoic acid) in an amount of at least 0.5% by weight of the composition.

18. An emulsion composition according to claim 17 wherein the poly(2-propenal, 2-propenoic acid) is present in an amount of from 0.5 to 5% by weight of the composition.

19. A process for preparing an emulsion composition comprising dissolving a poly(2-propenal, 2-propenoic acid) in an aqueous solution, mixing the aqueous phase with a polymeric oil phase in the presence of a polymeric emulsifier to provide an emulsion composition according to claim 1.

## Patentansprüche

1. Emulsionszusammensetzung für topische Anwendungen, mit:
(a) 10 bis 60 Gew.-% einer polymeren Ölphase mit einem mittleren Molekulargewicht von mindestens 1000;
(b) 1 bis 15 Gew.-% einer polymeren Emulgatorkomponente mit einem mittleren Molekulargewicht von mindestens 1000;
(c) 30 bis 90 Gew.-% einer wässerigen Phase; und
(d) mindestens 0,005 Gew.-% eines polymeren antimikrobiellen Mittels auf der Basis eines Poly(2-Propenal, 2-Propensäure)-Polymers, wobei das polymere antimikrobielle Mittel ein mittleres Molekulargewicht von mindestens 1000 hat;
wobei mindestens 95 Gew.-% der organischen Komponenten der Zusammensetzung ein mittleres Molekulargewicht von mindestens 1000 haben.

2. Emulsionszusammensetzung nach Anspruch 1, wobei das Poly(2-Propenal, 2-Propensäure)-Polymer durch Erwärmen in einer wässrigen Lösung von einem oder mehreren Polyethylenglykolen, Polyolen und Alkanolen aktiviert worden ist.

3. Emulsionszusammensetzung nach Anspruch 1, wobei die polymere Ölphase ein oder mehrere Polymere aufweist, die aus Polyalkylenglykolen, Organosilanpolymer (Polycarbosilanen), Polyvinylalkoholen, Polyvinylestern, Polyvinylpyrrolidonen, Alkoxypolyethylenglykolen, Polysilanen, Styrol-Acrylat-Copolymer, Polysacchariden, Copolymeren davon und Gemischen davon ausgewählt werden.

4. Emulsionszusammensetzung nach Anspruch 1, wobei die polymere Ölphase ein oder mehrerer Polymere aufweist, die aus Polyorganosiloxanen ausgewählt werden.

5. Emulsionszusammensetzung nach Anspruch 1, wobei der polymere Emulgator einen oder mehrere Emulgatoren aufweist, die aus Polyoxyalkylenemulgatoren und ethoxylierten Polysiloxanemulgatoren ausgewählt werden.

6. Emulsionszusammensetzung nach Anspruch 1, wobei der polymere Emulgator einen primären Emulgator, der einen oder mehrere ethoxylierte Polysiloxanemulgatoren enthält, und einen sekundären Emulgator aufweist, der einen oder mehrere Polyoxyalkylenemulgatoren enthält.

7. Emulsionszusammensetzung nach Anspruch 5, wobei der primäre Emulgator einen oder mehrere Emulgatoren enthält, die aus ethoxyliertem Polyacrylmethicon-Copolymer, Natrium-PG-Propyldimethiconthiosulfat-Copolymer und Cetyldimethicon-Copolyol ausgewählt werden.

8. Emulsionszusammensetzung nach Anspruch 5, wobei der sekundäre Emulgator einen oder mehrere Emulgatoren aufweist, die aus Polyethylenglykolethern umd polyethoxylierten Fettalkoholen ausgewählt werden.

9. Emulsionszusammensetzung nach Anspruch 5, wobei der primäre Emulgator in einer Menge von 1 bis 10 Gew.-% der Zusammensetung und der sekundäre Emulgator in einer Menge von 0,5 bis 5 Gew.-% der Zusammensetzung bereitgestellt wird.

10. Emulsionszusammensetzung nach Anspruch 1, wobei die Zusammensetzung 0,5 bis 5 Gew.-% eines Polymerfilmbildners enthält.

11. Emulsionszusammensetzung nach Anspruch 10, wobei der Polymerfilmbildner aus PVP/Polycarbomoylpolyglykolester, PVP/Eicosen-Copolymer, PVP/Dimethiconylacrylat/Polycarbamyl/Polyglykolester und Gemischen davon ausgewählt wird.

12. Emulsionszusammensetzung nach Anspruch 1, wobei die Zusammensetzung einen anorganischen oder polymeren Stabilisator enthält.

13. Emulsionszusammensetzung nach Anspruch 12, wobei der Stabilisator Magnesiumaluminiumsilikat ist.

14. Emulsionszusammensetzung nach Anspruch 1 zur Verwendung als Sonnenschutzmittel und ferner mit mindestens einem partikelförmigen Sonnenschutzmittel, das aus Zinkoxid und Titandioxid ausgewählt wird.

15. Emulsionszusammensetzung nach Anspruch 14, wobei das partikelförmige Sonnenschutzmittel in einer Menge von 0,5 bis 50 Gew.-% der Zusammensetung bereitgestellt wird.

16. Emulsionszusammensetzung nach Anspruch 15, wobei die Zusammensetzung 0,005 bis 2 Gew.-% eines Poly(2-Propenal, 2-Propensäure)-Polymers aufweist.

17. Emulsionszusammensetzung nach Anspruch 1 zur Verwendung als antiseptisches Hautmittel, mit einem Poly(2-Propenal, 2-Propensäure)-Polymer in einer Menge von mindestens 0,5 Gew.-% der Zusammensetzung.

18. Emulsionszusammensetzung nach Anspruch 17, wobei das Poly(2-Propenal, 2-Propensäure)-Polymer in einer Menge von 0,5 bis 5 Gew.-% der Zusammensetzung bereitgestellt wird.

19. Verfahren zum Herstellen einer Emulsionszusammensetzung mit den Schritten:
Lösen eines Poly(2-Propenal, 2-Propensäure)-Polymers in einer wässerigen Lösung;
Mischen der wässerigen Phase mit einer polymeren Ölphase bei Anwesenheit eines polymeren Emulgators zum Bereitstellen einer Emulsionszusammensetzung nach Anspruch 1.

## Revendications

1. Une composition en émulsion destinée à une application topique comprenant :
(a) de 10 à 60 % en poids de phase huileuse polymère ayant un poids moléculaire moyen d'au moins 1 000 ;
(b) de 1 à 15 % en poids de composant émulsifiant polymère de poids moléculaire moyen d'au moins 1 000 ;
(c) de 30 à 90 % en poids d'une phase aqueuse ; et
(d) au moins 0,005 % en poids d'antimicrobien polymère poly(2-propénal, acide 2-propénoïque), ledit antimicrobien polymère ayant un poids moléculaire moyen d'au moins 1 000,
où au moins 95 % en poids des composants organiques de la composition ont un poids moléculaire moyen d'au moins 1 000.

2. Une composition en émulsion selon la revendication 1 où le poly(2-propénal, acide 2-propénoïque) a été activé en le chauffant dans une solution aqueuse d'un ou de plusieurs polyéthylènes glycols, polyols et alcanols.

3. Une composition en émulsion selon la revendication 1 où la phase huileuse polymère comprend un ou plusieurs polymères sélectionnés dans le groupe consistant en polyalkylènes glycols, polymère organosilane (polycarbosilanes), alcools polyvinyliques, esters polyvinyliques, polyvinylpyrrolidones, alkoxypolyéthylènes glycols, polysilanes, copolymère de styrène-acrylate, polysaccharides, copolymères de ceux-ci et mélanges de ceux-ci.

4. Une composition en émulsion selon la revendication 1 où la phase huileuse polymère comprend un ou plusieurs polymères sélectionnés dans le groupe consistant en polyorganosiloxanes.

5. Une composition en émulsion selon la revendication 1 où l'émulsifiant polymère inclut un ou plusieurs émulsifiants sélectionnés dans le groupe d'émulsifiants polyoxyalkylènes et d'émulsifiants polysiloxanes éthoxylés.

6. Une composition en émulsion selon la revendication 1, où l'émulsifiant polymère comprend un émulsifiant primaire comprenant un ou plusieurs émulsifiants polysiloxanes éthoxylés et un émulsifiant secondaire comprenant un ou plusieurs émulsifiants polyoxyalkylènes.

7. Une composition en émulsion selon la revendication 5, où l'émulsifiant primaire comprend un ou plusieurs émulsifiants sélectionnés dans le groupe consistant en copolymère polyacrylméthicone éthoxylé, copolymère sodium PG-propyldiméthicone thiosulfate et cétyl diméthicone copolyol.

8. Une composition en émulsion selon la revendication 5, où l'émulsifiant secondaire comprend un ou plusieurs émulsifiants sélectionnés dans le groupe consistant en éthers de polyéthylène glycol et alcools gras polyéthoxylés.

9. Une composition en émulsion selon la revendication 5, où l'émulsifiant primaire est présent dans une quantité allant de 1 à 10 % en poids de la composition et l'émulsifiant secondaire est présent dans une quantité allant de 0,5 à 5 % en poids de la composition.

10. Une composition en émulsion selon la revendication 1 où la composition inclut de 0,5 à 5 % d'un agent filmogène polymère.

11. Une composition en émulsion selon la revendication 10 où l'agent filmogène polymère est sélectionné dans le groupe consistant en PVP / ester de polycarbomoylpolyglycol, copolymère de PVP / d'éicosène, PVP / diméthiconylacrylate / polycarbamyl / ester de polyglycol, et mélanges de ceux-ci.

12. Une composition en émulsion selon la revendication 1, où la composition comprend un agent stabilisant inorganique ou polymère.

13. Une composition en émulsion selon la revendication 12, où l'agent stabilisant est du silicate de magnésium et d'aluminium.

14. Une composition en émulsion selon la revendication 1 destinée à être utilisée comme protection solaire et comprenant de plus au moins un agent de protection solaire particulaire sélectionné parmi l'oxyde de zinc et le dioxyde de titane.

15. Une composition en émulsion selon la revendication 14 où l'agent de protection solaire particulaire est présent dans une quantité allant de 0,5 % à 50 % en poids de la composition.

16. Une composition en émulsion selon la revendication 15 où la composition comprend un poly(2-propénal, acide 2-propénoïque) dans une quantité allant de 0,005 à 2 % en poids.

17. Une composition en émulsion selon la revendication 1 destinée à être utilisée comme antiseptique cutané comprenant du poly(2-propénal, acide 2-propénoïque) dans une quantité d'au moins 0,5 % en poids de la composition.

18. Une composition en émulsion selon la revendication 17 où le poly(2-propénal, acide 2-propénoïque) est présent dans une quantité allant de 0,5 à 5 % en poids de la composition. ,

19. Une méthode pour préparer une composition en émulsion comprenant la dissolution d'un poly(2-propénal, acide 2-propénoïque) dans une solution aqueuse, le mélange de la phase aqueuse avec une phase huileuse polymère en présence d'un émulsifiant polymère pour fournir une composition en émulsion selon la revendication 1.
